(19) Europäisches Patentamt
European Patent Office
Office européen des brevets

(11) **EP 2 143 704 A1**

(12) **EUROPEAN PATENT APPLICATION**

(43) Date of publication:
**13.01.2010 Bulletin 2010/02**

(51) Int Cl.:
*C07C 51/25* (2006.01)        *C07C 57/055* (2006.01)
*C07C 253/26* (2006.01)

(21) Application number: **09155567.2**

(22) Date of filing: **19.03.2009**

(84) Designated Contracting States:
**AT BE BG CH CY CZ DE DK EE ES FI FR GB GR HR HU IE IS IT LI LT LU LV MC MK MT NL NO PL PT RO SE SI SK TR**
Designated Extension States:
**AL BA RS**

(30) Priority: **10.07.2008 US 134458 P**

(71) Applicant: **Rohm and Haas Company Philadelphia, PA 19106-2399 (US)**

(72) Inventors:
• **Han, Scott**
  **Lawrenceville, NJ 08648 (US)**
• **Ruettinger, Wolfgang**
  **East Windsor, NJ 08520 (US)**
• **Sevon, Douglass, W.**
  **Fairless Hills, PA 19030 (US)**

(74) Representative: **Kent, Venetia Katherine**
  **Patent Outsourcing Limited**
  **1 King Street**
  **Bakewell**
  **Derbyshire DE 45 1DZ (GB)**

(54) **An integrated process for preparing a carboxylic acid from an alkane**

(57) The present invention relates to an integrated process for producing unsaturated carboxylic acids from the corresponding $C_2$-$C_4$ alkane. The process begins with integrated catalytic reactions comprising endothermic soft oxidant conversion (SOC) and exothermic selective hydrogen combustion (SHC) which cumulatively convert a $C_2$-$C_4$ alkane to its corresponding $C_2$-$C_4$ alkene in a product stream substantially free of hydrogen. Heat from the exothermic selective hydrogen combustion reaction zone is recovered and recycled back upstream to the endothermic soft oxidant conversion reaction zone. The hydrogen-free alkene products of the thermally integrated SOC/SHC reactions are then provided to a catalytic vapor phase partial oxidation process for conversion of the alkene to the corresponding unsaturated carboxylic acid or nitrile. Unreacted alkene and carbon dioxide are recovered from the oxidation product stream and recycled back to the thermally integrated soft oxidant conversion reactions.

EP 2 143 704 A1

**Description**

Government Interest

This invention was made with Government support under Instrument No. DE-FC36-O4G01427 awarded by the United States Department of Energy. The Government has certain rights in this invention.

Field of the Invention

The present invention relates to an integrated process for preparing a carboxylic acid from its corresponding alkane utilizing an energy efficient process in which soft oxidant conversion and selective hydrogen combustion collectively convert the alkane to its corresponding alkene, followed by partial oxidation of the alkene to produce the carboxylic acid.

Background of the Invention

Well-known commercial processes for the production of monomers, such as unsaturated carboxylic acids and unsaturated nitriles, typically start with one or more alkenes and convert them, by catalytic vapor phase oxidation, to the desired monomer products. In view of the pressures exerted by competition in the industry, and the price difference between alkanes and their corresponding alkenes, such as propane and propene, respectively, efforts are being made to develop processes in which an alkane is used as the starting material to, ultimately, produce the desired monomers at a lower overall cost.

One process modification, which has enjoyed some success in commercial industry, is to simply add an upstream reaction stage in which an alkane is first converted to the corresponding alkene, in the presence of a suitable catalyst. The resulting alkene (e.g., propene) is then fed to the customary oxidation reaction stages, for oxidation of the alkene (e.g., first to acrolein and then to the desired monomer product, as in the two-step oxidation of propene to form acrylic acid). For example, both European Patent Application No. EP0117146 and US Patent No. 5,705,684 describe multi-stage catalytic processes for converting an alkane (propane) to the corresponding unsaturated carboxylic acid (acrylic acid) which includes an initial alkane-to-alkene conversion stage having one or more suitable catalysts to produces a product stream comprising alkene, which is fed to one or more downstream oxidation stages. Various catalysts and methods are known to catalyze conversion of alkanes to their corresponding alkenes.

For example, various mixed metal oxide catalysts have been shown to be useful for oxidative dehydrogenation of propane to produce propene. See, U.S. Patent Nos. 4,046,833 and 6,239,325, U.S. Patent Application Publication Nos. 2005/0124840 and 2005/0085678), as well as Zhagorigetu, B.; Kieffer, R.; Hinderman J.-P., "Oxidative Dehydrogenation of Propane on Rare Earth Vanadates. Influence of the Presence of $CO_2$ in the feed." Studies in Surface Science and Catalysis, 1996, 101, 1049-1058). However, since it is exothermic, when an oxidative dehydrogenation process is operated continuously, excess heat must be continuously removed, which increases capital and operating expenditures. Another disadvantage of oxidative dehydrogenation is that selectivity to alkene tends to decrease when the process is operated at higher, commercially useful alkane conversion rates. Thus, in practice, these processes tend to be operated at lower conversion rates (well below 100%), which limits their product yield capacity and generally renders them economically unsuitable for use in commercial-scale processes.

Other catalysts are known to catalyze the non-oxidative dehydrogenation of an alkane, in the presence of a "weak" oxidant, such as steam or carbon dioxide, to form the corresponding alkene. So as not to confuse the terms oxidative dehydrogenation, which requires oxygen (a strong oxidant) and endothermic dehydrogenation, which uses a soft oxidant in place of oxygen, "endothermic dehydrogenation" will hereinafter be referred to as non-oxidative dehydrogenation, or soft oxidant conversion. Some soft oxidant conversion catalysts perform better in the absence of oxygen, while others tolerate the presence of minor amounts of oxygen, along with the weak oxidant, without significant loss of activity. Supported vanadium-based catalysts, promoted with Li, Na, K or Mg, have been shown to dehydrogenate ethylbenzene in the presence of a "soft oxidant," i.e., carbon dioxide, to produce styrene with selectivities of about 98-99%, in the absence of oxygen (Li, X.-H.; Li, W.-Y.; Xie, K.-C., "Supported Vanadia Catalysts for Dehydrogenation of Ethylbenzene with $CO_2$," Catalyst Letters, December 2005, Vol. 105, Nos. 3-4). Carbon dioxide was provided in varying amounts by Dury, et al. in 2002 to the oxidative dehydrogenation of propane to form propene in the presence of nickel-molybdenum-based catalysts, and found to increase conversion (by about 18 - 28%) but decrease selectivity (Dury, F.; Gaigneaux, E.M., Ruiz, P., "The Active Role of $CO_2$ at Low Temperature in Oxidation Processes: The Case of the Oxidative Dehydrogenation of Propane on NiMoO4 catalysts," Applied Catalysis A: General 242 (2003), 187-203). Dury et al. demonstrated that, contrary to the traditional expectation that carbon dioxide is inert in dehydrogenation reactions, carbon dioxide actively participates in the dehydrogenation of propane, even in the absence of oxygen. Takehira, et al. tested the activities of various metal oxide catalysts (Cr, Ga, Ni, V, Fe, Mn and Co) supported on silicon-containing support materials, including mesoporous MCM-41, Cab-O-Sil, and silicon oxide, and found that the Cr-based catalyst supported on MCM-41 provided the best results for dehydrogenation of propane, in the presence of carbon dioxide, to form propene. Takehira, K.; Oishi, Y.; Shishido, T.; Kawabata, T.; Takaki, K.; Zhang, Q.; and Wang, Y., "CO2 Dehydrogenation of Propane over Cr-MCM-41 Catalyst," Studies in Surface Science and Catalysis, 2004, 153, 323-328.

Non-oxidative dehydrogenation reactions are endothermic and, therefore require addition of heat to the process. One way of providing heat to the non-oxidative dehydrogenation process is to recover heat from another, separate, process, or even from a related downstream process, such as a process step in which the alkene produced by the non-oxidative dehydrogenation is utilized, and recycle that heat back to the non-oxidative dehydrogenation reaction zone.

Provision of heat to non-oxidative dehydrogenation processes has also been accomplished by burning (i.e., combusting) a hydrocarbon fuel, often different than the alkane to be dehydrogenated, with oxygen in a furnace or other vessel, resulting in increased costs due to increased initial capital investment and ongoing fuel consumption. European Patent Application Publication No. EP 1112241 ("EP '241 ") describes a process which addressed this issue. Rather than burning a separate fuel to produce heat, the disclosed process involves combusting a portion of the alkane which is to be dehydrogenated, with oxygen, in the presence of a suitable combustion catalyst, to produce a heated stream containing the products of combustion (i.e., carbon oxides and water), unconsumed oxygen and unconsumed alkane. The heated stream is fed directly to an endothermic catalytic dehydrogenation reaction stage where the unreacted alkane is converted to the corresponding alkene in the presence of a suitable dehydrogenation catalyst.

Thus, in the process of EP '241, the need to burn a separately provided hydrocarbon fuel to preheat the alkane feed is avoided. However, a portion of the alkane reactant is consumed, which leaves less available for conversion to the desired product in the dehydrogenation stage. Furthermore, products of combustion are incidentally formed, which increases the amount of unwanted by-products, without any contribution to the quantity of the desired alkene product. In fact, when a portion of the alkane reactant itself is burned, as taught by these sources, a diminished amount of alkane remains available for the dehydrogenation reaction and less of the desired alkene product is produced.

Additionally, U.S. Patent No. 4,788,371 describes a process for steam dehydrogenation of hydrocarbons in the vapor phase with simultaneous oxidative reheating of the intermediate products by selective hydrogen combustion. The process utilizes a single catalyst composition to accomplish both the selective oxidation and steam dehydrogenation reactions. The particular catalyst employed comprises a Group VIII noble metal component, a Group IA and/or a Group IIA component and may contain among other modifiers a Group IIIA or IVA metal, and a halogen component. The catalytic components are supported on an inorganic substrate such as alumina. More particularly, the catalyst composition catalyzes the dehydrogenation of a hydrocarbon, which is an endothermic reaction, and also catalyzes the oxidation (combustion) of the dehydrogenation byproduct hydrogen to produce heat to sustain further dehydrogenation of the hydrocarbon. Reaction temperatures were reported as ranging between 400°C to 900°C, depending upon the particular hydrocarbon reactant involved.

Grasselli, et al., have reported successful non-oxidative dehydrogenation of light alkane hydrocarbons in combination with selective hydrogen combustion, using two different catalyst compositions which are either arranged in series of successive catalyst beds, or mixed together in a single catalyst bed. See Grasselli, et al., "Catalytic dehydrogenation (DH) of light parrafins combined with selective hydrogen combustion (SHC) I. DH -> SHC -> DH catalysts in series (co-fed process mode)," Applied Catalysis A: General 189 (1999), 1-8, and Grasselli, et al., "Catalytic dehydrogenation (DH) of light parrafins combined with selective hydrogen combustion (SHC) II. DH+SHC catalysts physically mixed (redox process mode)," Applied Catalysis A: General 189 (1999), 9-14, respectively. No oxygen was provided to the reaction stage containing the non-oxidation dehydrogenation catalyst, which comprised zeolite-supported platinum and tin, while the selective hydrogen combustion catalysts comprised an oxide of a metal selected from indium, bismuth, lead and zinc, supported on zirconium, alumina or silica substrates.

An autothermal "hybrid" process has been developed for conversion of an alkane to its corresponding alkene in a thermally integrated two-stage process which is described in U.S. Patent Application Publication No. US2008/_____ (**DN A01855,** US Ser 11/901,102, filed Sept 13, 2007). In particular, an alkane and oxygen are provided to a first reaction stage wherein a portion of the alkane is converted, by exothermic oxidative dehydrogenation in the presence of an upstream oxidative dehydrogenation catalyst and oxygen, to form an intermediate heated product stream containing heat, a small amount of the corresponding alkene and the remaining unreacted alkane. This intermediate heated product stream is then provided to a second stage wherein the remaining unreacted alkane is converted in an endothermic non-oxidative dehydrogenation reaction in the presence of a catalyst and a weak oxidant, such as carbon dioxide, to form a cumulative product stream containing additional amounts of the corresponding alkene, as well as carbon oxides, water and hydrogen. As already mentioned, the hydrogen present in the endothermic second stage product stream is known to contribute to unwanted side reactions and commensurate decreases in the yield of the desired partial oxidation products and, therefore, must be removed from the product stream prior to providing it to downstream oxidation process steps. As discussed in the background section of U.S. Patent Application Publication No. US2008/_____, chromium-based catalysts, aluminum-based catalysts, and vanadium-based catalysts, among others, have been found useful for catalytic conversion of one or more $C_2$-$C_4$ alkanes to form the corre-

sponding $C_2$-$C_4$ alkenes and hydrogen in the presence of a soft oxidant, e.g., carbon dioxide, in the absence of oxygen (i.e., "soft oxidant conversion catalysts"). Furthermore, while a Cr-based catalyst supported on silica provided superior results for conversion of propane, in the presence of carbon dioxide, to form propene, it was also noted that water present in the intermediate heated product stream irreversibly deactivates the chromium-based catalyst in the endothermic second stage.

Additionally, as described in U.S. Patent Application Publication No. US2008/_____ **(DN A01963,** US Provisional Ser No 61/009,118, filed December 26, 2007**)**, an integrated process for preparation of a carboxylic acid from its corresponding alkane has been developed wherein the alkene-containing product stream from the aforesaid autothermal "hybrid" is subsequently subjected to partial oxidation of the alkene to produce the carboxylic acid.

Accordingly, notwithstanding the work conducted to date in this field, industry continues to grapple with the aforesaid problems of increasing overall production of useful monomers such as unsaturated carboxylic acids and nitriles, while minimizing the costs of such production. Increasing alkene selectivity and yield during dehydrogenation of lower alkanes to their corresponding alkenes is necessary, as well as addressing the various issues raised when directly coupling such alkene production methods with processes to convert the alkene to its corresponding unsaturated carboxylic acids on a commercial scale. It is believed that the process of the present invention address these issues and achieves an integrated and economical process for production of unsaturated carboxylic acids from the corresponding alkanes.

Summary of the Invention

The present invention provides a process for producing an unsaturated carboxylic acid or an unsaturated nitrile from the corresponding $C_2$-$C_4$ alkane. More particularly, the process comprises converting a $C_2$-$C_4$ alkane to its corresponding $C_2$-$C_4$ alkene by providing a $C_2$-$C_4$ alkane, a weak oxidant and heat to an endothermic reaction zone comprising an upstream catalyst to produce an intermediate product gas comprising at least the corresponding $C_2$-$C_4$ alkene and hydrogen; and then converting at least a portion of the hydrogen in the intermediate product gas to water by providing the intermediate product gas and oxygen to an exothermic reaction zone to produce a cumulative product gas which comprises the corresponding $C_2$-$C_4$ alkene, water, carbon oxides and heat. Furthermore, the process requires recovering at least a portion of the heat from the cumulative product gas and providing the recovered heat to the endothermic reaction zone, wherein at least a portion of the heat provided in the initial converting step comprises the recovered heat and wherein a cooled cumulative product gas is produced. The cooled cumulative product gas comprises no more than about 5% by weight of hydrogen, based on the total weight of the cumulative product gas.

Thereafter, the $C_2$-$C_4$ alkene of the cooled cumulative product gas is oxidized to produce an oxidation product stream comprising the corresponding unsaturated carboxylic acid or nitrile, in the presence of at least one catalyst capable of facilitating the oxidizing reaction. The oxidation product stream is separated into an aqueous oxidation product stream comprising the corresponding unsaturated carboxylic acid or nitrile, and a gas stream comprising unreacted $C_2$-$C_4$ alkane and unreacted carbon dioxide. The process further involves drying the gas stream and separating the unreacted $C_2$-$C_4$ alkane and unreacted carbon dioxide therefrom and recycling the separated unreacted $C_2$-$C_4$ alkane and unreacted carbon dioxide to the initial step of converting a $C_2$-$C_4$ alkane in an endothermic reaction zone. The weak oxidant may comprise carbon dioxide.

In one embodiment the upstream catalyst may be a soft oxidant conversion catalyst which catalyzes the endothermic conversion of the $C_2$-$C_4$ alkane to the corresponding $C_2$-$C_4$ alkene in the presence of the weak oxidant. The soft oxidant conversion catalyst may comprise: chromium or chromium oxide; optionally, one or more metals selected from the group consisting of Mo, W, V, Ga, Mg, Ni and Fe; and, optionally, one or more metals selected from the group consisting of Ag, V and Ga. The soft oxidant conversion catalyst may also comprise a support material

Furthermore, the downstream catalyst may be a selective hydrogen combustion catalyst which catalyzes the exothermic conversion of hydrogen to water in the presence of oxygen. The selective hydrogen combustion catalyst may comprise at least one catalyst composition selected from the group consisting of: A) a catalyst comprising a noble metal selected from the group consisting of platinum and palladium, and, optionally, another metal selected from the group consisting of tin and iridium; and B) a catalyst comprising an oxide of indium or bismuth, and, optionally, another metal selected from the group consisting of molybdenum. The selective hydrogen combustion catalyst may also comprise a support material.

In another embodiment of the process of the present invention, the $C_2$-$C_4$ alkane comprises propane, the corresponding $C_2$-$C_4$ alkene comprises propene, the weak oxidant comprises carbon dioxide, and the corresponding unsaturated carboxylic acid is acrylic acid. For example, the $C_2$-$C_4$ alkene of the recovery stream is oxidized by a two-step vapor phase catalytic oxidation wherein a first oxidation reaction zone comprises a first oxidation catalyst capable of catalyzing the conversion of the $C_2$-$C_4$ alkene to the corresponding aldehyde, and a second oxidation reaction zone, positioned downstream of the first oxidation reaction zone and comprising a second oxidation catalyst, different from the first oxidation catalyst and capable of catalyzing the conversion of the corresponding aldehyde to the corresponding unsaturated carboxylic acids or nitriles.

Brief Description of the Drawings

A more complete understanding of the present invention will be gained from the embodiments discussed hereinafter and with reference to the accompanying drawing, wherein:

Figure 1 is a generalized schematic representation of the process of the present invention; and
Figure 2 is a schematic representation of an embodiment of the process of the present invention.

Detailed Description of the Invention

The following definitions and meanings are provided for clarity and will be used hereinafter.

The term "hydrocarbon" means a compound which comprises at least one carbon atom and at least one hydrogen atom.

As used herein, the term "$C_2$ to $C_4$ alkane" means a straight chain or branched chain alkane having from 2 to 4 carbons atoms per alkane molecule, for example, ethane, propane and butane, which are typically in the vapor phase at ordinary temperatures and pressures (e.g., at least 10°C and 1 atmosphere). Accordingly, the term "$C_2$ to $C_4$ alkene" means a straight chain or branched chain alkene having from 2 to 4 carbons atoms per alkene molecule, for example, ethane, propene and butene.

The term "corresponding $C_2$-$C_4$ alkene" means the alkene having the same number of carbon atoms per alkene molecule as the particular $C_2$-$C_4$ alkane under discussion.

Furthermore, as used herein, the term "$C_2$ to $C_4$ alkanes and alkenes" includes at least one of the aforesaid $C_2$-$C_4$ alkanes, as well as its corresponding $C_2$-$C_4$ alkene. Similarly, when used herein in conjunction with the terms "$C_2$ to $C_4$ alkane", or "$C_2$ to $C_4$ alkene", or "$C_2$ to $C_4$ alkanes and alkenes", the terminology "a mixture thereof," means a mixture that includes at least one of the aforesaid alkanes having from 2 to 4 carbons atoms per alkane molecule, and the alkene having the same number of carbons atoms per alkene molecule as the alkane under discussion, for example, without limitation, a mixture of propane and propene, or a mixture of n-butane and n-butene.

An "inert" material, sometimes also referred to as a "diluent," is any material which is substantially inert, i.e., does not participate in, is unaffected by, and/or is inactive, in the particular reaction of concern. For example, nitrogen is generally considered to be inert in reactions that convert alkanes to their corresponding alkenes. As a more specific example, nitrogen is inert in dehydrogenation reactions that produce propene from propane. In the context of catalysts, where a mixed metal oxide catalyst useful in oxidation reactions is supported by a zirconium-based material, the zirconium-based material is considered to be inert and, as such, is understood to not directly affect, and not be directly affected by, the oxidation reaction being catalyzed by the mixed metal oxide catalyst. (Rather, without being bound by theory, it is believed that some support materials, such as zirconium, directly interact with the catalyst, which in turn may affect the conversion, selectivity, etc., of the oxidation reaction.)

The efficacy of chemical reaction processes, including those discussed herein, may be characterized and analyzed using the terms "feed conversion," "selectivity" to a particular product, and "product yield." These terms are used hereinafter and will have the following standard meanings.

The feed conversion, or simply "conversion", is the percentage of the total moles of feed (e.g., $C_3$ to $C_5$ alkanes and alkenes, such as propane and propene, or a mixture thereof) that have been consumed by the reaction, regardless of what particular products were produced, and is generally calculated as follows:

$$\text{feed conversion (\%)} = \frac{\text{moles of feed converted}}{\text{moles of feed supplied}} \times 100$$

The selectivity to a particular product, or simply "selectivity," is the percentage of the percentage of the total moles of feed (e.g., $C_3$ to $C_5$ alkanes, such as ethane, propane, and propene, or a mixture thereof) that have been consumed by the reaction, i.e., the portion of the feed that has been consumed was actually converted to the desired product, regardless of other products. Selectivity is generally calculated as follows:

$$\text{selectivity (\%)} = \frac{\text{moles of desired product produced}}{\text{moles of feed converted}} \times \frac{\text{number of carbon atoms in product}}{\text{number of carbon atoms in feed}} \times 100$$

The product yield, or simply "yield," is the percentage of the theoretical total moles of the desired product (alkene) that would have been formed if all of the feed had been converted to that product (as opposed to unwanted side products, e.g. acetic acid and $CO_x$ compounds), and is generally calculated as follows:

$$\text{product yield (\%)} = \frac{\text{moles of product produced}}{\text{moles of feed supplied}} \times \frac{\text{number of carbon atoms in product}}{\text{number of carbon atoms in feed}} \times 100$$

The term "oxygen-containing gas," as used herein, means any gas comprising from 0.01% up to 100% oxygen or oxygen-containing compounds, including for example, without limitation: air, oxygen-enriched air, nitrous oxide, nitrogen dioxide, pure oxygen, mixtures of pure oxygen or oxygen-containing compounds with at least one inert gas, such as nitrogen, and mixtures thereof. Although the oxygen containing gas may be pure oxygen gas, it is usually more economical to use an oxygen containing gas, such as air, when purity is not particularly required.

"Dehydrogenation," as used herein, means a chemical reaction in which one or more hydrogen atoms are eliminated from a hydrocarbon having at least 2 carbon atoms. Dehydrogenation is used, for example, to convert alkanes (such as, ethane, propane, and butane) into olefins (such as ethylene, propylene, and butenes, respectively). Molecular hydrogen is often a product of dehydrogenation reactions, along with the desired olefin product. In particular, "oxidative dehydrogenation" means the dehydrogenation of a hydrocarbon having at least 2 carbon atoms in the presence of oxygen and accompanied by the production of heat.

"Selective hydrogen combustion," as used herein, means a chemical process which converts hydrogen, in the presence of oxygen, to produce water and heat.

Generally, "soft oxidant conversion," as used hereinafter, means a chemical reaction in which one or more hydrogen atoms are eliminated from a hydrocarbon having at least 2 carbon atoms, and which consumes heat. Thus, soft oxidant conversion reactions require heat to be supplied from a source external to the non-oxidative dehydrogenation reaction. Since hydrogen is removed from the hydrocarbon, soft oxidant conversion may also be referred to as "non-oxidative dehydrogenation." More particularly, as used herein, soft oxidant conversion refers to the catalytic conversion of a $C_2$-$C_4$ alkane to its corresponding $C_2$-$C_4$ alkene in the presence of a soft oxidant, such as carbon dioxide, and a soft oxidant conversion catalyst.

As used hereinafter, a "soft oxidant conversion catalyst" is a catalyst composition which catalyzes catalytic conversion of one or more $C_2$-$C_4$ alkanes to form the corresponding $C_2$-$C_4$ alkenes and hydrogen in the presence of a soft oxidant, e.g., carbon dioxide, in the absence of oxygen.

The terms "cumulatively convert" and "cumulatively produce" are each used interchangeably to describe the desired end product(s) of a set of two or more chemical reactions relative to the initial starting materials, regardless of intermediate reaction mechanisms and products other than those intended.

Endpoints of ranges are considered to be definite and are recognized to incorporate within their tolerance other values within the knowledge of persons of ordinary skill in the art, including, but not limited to, those which are insignificantly different from the respective endpoint as related to this invention (in other words, endpoints are to be construed to incorporate values "about" or "close" or "near" to each respective endpoint). The range and ratio limits, recited herein, are combinable. For example, if ranges of 1-20 and 5-15 are recited for a particular parameter, it is understood that ranges of 1-5, 1-15, 5-20, or 15-20 are also contemplated and encompassed thereby.

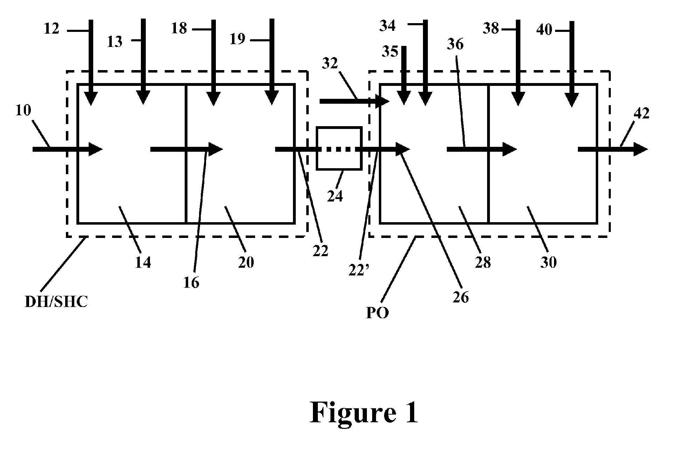

The process of the present invention involves sequential, thermally integrated endothermic soft oxidant conversion and exothermic selective hydrogen combustion reactions **SOC/SHC** (see Figure 1), which cumulatively convert a particular $C_2$-$C_4$ alkane to the corresponding $C_2$-$C_4$ alkene in a substantially hydrogen-free product stream, as well as downstream partial oxidation reactions **PO** (see Figure 1), which convert the $C_2$-$C_4$ alkene product to corresponding unsaturated carboxylic acids. Since the soft oxidant conversion and selective hydrogen combustion reactions **SOC/SHC** produce the corresponding $C_2$-$C_4$ alkene with greater overall thermal efficiency and lower hydrogen content, than the endothermic soft oxidant conversion reaction alone, the ultimate yield of corresponding unsaturated carboxylic acids from the subsequent partial oxidation reactions **PO** is also increased compared to conventional, known oxidation processes for producing unsaturated carboxylic acids from the corresponding $C_2$-$C_4$ alkenes. It is noted that, although the partial oxidation reactions are described hereinafter as a two-stage partial oxidation of alkane to its corresponding aldehyde, followed by partial oxidation of the aldehyde to the corresponding unsaturated carboxylic acid, it is possible for the partial oxidation reactions **PO** to be accomplished by other processes, such as use of a single catalyst composition which catalyzes the partial oxidation of an alkane, the corresponding alkene, or mixtures thereof to produce the corresponding unsaturated carboxylic acid.

The overall integrated process, as well as catalysts suitable for use therein, will first be described in general. Then a more detailed description is provided of an exemplary embodiment of the present invention, which is a combination of an endothermic soft oxidant conversion reaction and an exothermic selective hydrogen combustion reaction for converting propane to propene, followed by two-stage vapor phase partial oxidation of the propene to produce unsaturated carboxylic acid (i.e., acrylic acid), will be provided in the examples.

Notwithstanding the specificity of the exemplary embodiments, it will be appreciated and understood by persons of ordinary skill that the present invention is applicable to other types of reactions and products, and is subject to modifications and alterations, as necessary and desired, according to the ordinary skill and general knowledge of persons practicing in the relevant art. For example, the process of the present invention may be easily adapted by skilled persons to dehydrogenate a variety of hydrocarbons such as ethane and ethyl benzene.

Catalysts suitable for use as upstream catalysts in the endothermic reaction zone of the **SOC/SHC** process are not particularly limited and include, but are not limited to, soft oxidant conversion catalysts recommended in the prior art for catalyzed soft oxidant conversion of hydrocarbons, in the gas phase and in the presence of a weak oxidant, to form molecular hydrogen.

Similarly, catalysts suitable for use as downstream, catalysts in the exothermic reaction zone of the SOC/SHC process are not particularly limited and include, but are not limited to, selective hydrogen combustion catalysts recommended in the prior art for selective combustion of hydrogen, in the gas phase and in the presence of oxygen, to form water and heat.

The upstream and downstream catalysts may be prepared by any suitable method known in the art, now or in the future. For example, the catalyst can be prepared by incipient wetness impregnation, chemical vapor deposition, hydrothermal synthesis, salt melt method, co-precipitation, and other methods. As will be discussed in further detail hereinafter, catalysts which are active for exothermic or endothermic conversion of $C_2$-$C_4$ alkanes to produce the corresponding $C_2$-$C_4$ alkenes typically comprise one or more metals and/or metal oxides. In addition, either or both of the upstream and downstream catalysts may be promoted, for example, with suitable metals or metal oxides.

Furthermore, either or both of the upstream and downstream catalysts may further comprise support material. The catalyst materials may be applied to the support by any method known in the art and at any time including, but not limited to, during preparation of the catalyst material, before or after calcination, and even before or after addition of a promoter. Typical and suitable support materials include, but are not limited to: magnesium oxide, zirconia, stabilized zirconia, zirconia stabilized alumina, yttrium stabilized zirconia, calcium stabilized zirconia, alumina, titania, silica, magnesia, nitrides, silicon carbide, cordierite, cordierite-alpha alumina, alumina-silica magnesia, zircon silicate, magnesium silicates, calcium oxide, silica-alumina, alumina-zirconia, alumina-ceria, and combinations thereof. Additionally, suitable catalyst supports may comprise rare earth metal oxides, mixed metal oxides, mesoporous materials, refractory materials, and combinations thereof. The support may be modified, stabilized, or pretreated in order to achieve the proper structural stability desired for sustaining the operating conditions under which the catalysts will be used.

The support can be in the shape of wire gauzes, monoliths, particles, honeycombs, rings, and others. Where the support is in the form of particles, the shape of the particles is not particularly limited and may include granules, beads, pills, pellets, cylinders, trilobes, spheres, irregular shapes, etc.

Monoliths typically comprise any unitary piece of material of continuous manufacture, such as, for example, pieces of metal or metal oxide, foam materials, or honeycomb structures. It is known in the art that, if desired, a reaction zone may comprise two or more such catalyst monoliths stacked upon one another. For example, the catalyst can be structured as, or supported on, a refractory oxide "honeycomb" straight channel extrudate or monolith, made of cordierite or mullite, or other configuration having longitudinal channels or passageways permitting high space velocities with a minimal pressure drop.

Furthermore, the catalyst material may be deposited as washcoats on the monolithic support by methods known to people skilled in the art. Additionally, catalyst material may be combined with the monolithic support by depositing the support material as washcoats and, successively, impregnating the support material washcoats with the active catalyst material, such as, without limitation, chromium oxide or vanadium oxide, followed by calcination of the combined support and catalyst materials.

Monolithic supports may comprise stabilized zirconia (PSZ) foam (stabilized with Mg, Ca or Y), or foams of silica, α-alumina, cordierite, ceramics, titania, mullite, zirconium-stabilized α-alumina, or mixtures thereof. Monolithic supports may also be fabricated from metals and their alloys, such as, for example, aluminum, steel, fecralloy, hastalloy, and others known to persons skilled in the art. Additionally, other refractory foam and non-foam monoliths may serve as satisfactory supports. The promoter metal precursor and any base metal precursor, with or without a ceramic oxide support forming component, may be extruded to prepare a three-dimensional form or structure such as a honeycomb, foam or other suitable tortuous-path or straight-path structure.

In an exemplary embodiment, the upstream catalyst should be a soft oxidant conversion catalyst which catalyzes the endothermic conversion of a $C_2$-$C_4$ alkane, in the presence of a mild oxidant and in the absence of oxygen, to the corresponding $C_2$-$C_4$ alkene and hydrogen. The mild oxidant may be, for example, without limitation, carbon dioxide, steam, or a combination thereof. As discussed hereinabove, many such endothermic soft oxidant conversion catalysts are known and would be suitable for use the endothermic reaction zone in accordance with process of the present invention.

Persons of ordinary skill will be familiar with various soft oxidant conversion catalysts that may be successfully used in the endothermic reaction zone, in accordance with process of the present invention. Suitable categories of soft oxidant conversion catalysts include, but are not limited to: chromium-based catalysts, which may also comprise oxides of at least one metal selected from the group consisting of, for example, Mo, W, V, Ga, Mg, Ni, and Fe; as well as vanadium oxide-based catalysts, which may be promoted with Cr, Li, Na, K or Mg. For example, chromium-based catalysts which also comprise a metal selected from the group consisting of silver, vanadium and gallium, and which are supported on silica or alumina, are known to be particularly suitable soft oxidant conversion catalysts for use in the process of the present invention.

Also in an exemplary embodiment, the downstream catalyst should be a selective hydrogen combustion catalyst which catalyzes the exothermic combustion of hydrogen, in the presence of oxygen, to form water and heat. As discussed hereinabove, many such exothermic selective hydrogen combustion catalysts are known and would be suitable for use the exothermic reaction zone in accordance with process of the present invention. For example, supported platinum-based catalysts would be suitable for use in the exothermic reaction zone of the present invention.

Persons of ordinary skill will be familiar with various exothermic selective hydrogen combustion catalysts that may be successfully used in the exothermic reaction zone, in accordance with process of the present invention. Suitable categories of exothermic selective hydrogen combustion catalysts include, but are not limited to: oxides of metals such as indium, bismuth, lead and zinc, and catalysts comprising one or more Group VIII noble metals (such as platinum, palladium, iridium, rhodium, osmium and ruthenium) with one or more of rubidium, cesium, potassium, sodium, lithium and francium, as well as one or more of boron, gallium, indium, germanium, tin and lead. The selective hydrogen combustion catalyst may be supported on materials such as alumina, silica, zirconia, zeolites, other metal oxides, microporous materials, mesoporous materials, and refractory materials.

As will be easily recognized by skilled persons, there are many catalyst compositions suitable for use in the exothermic reaction zone in accordance with the present invention. For example, the exothermic selective hydrogen combustion catalyst may comprise platinum, supported on silica, with or without tin or indium. Another suitable selective hydrogen combustion catalyst would comprise a noble metal component comprising platinum or palladium, another component comprising tin and/or indium, and still another component comprising cesium and/or potassium, all supported on alumina or silica.

Referring now to the schematic representation of the process of the present invention provided in Figure 1, generally, a $C_2$-$C_4$ alkane 10 and a weak or mild oxidant 12, such as carbon dioxide, are contacted with an upstream catalyst (not shown per se) in an endothermic reaction zone 14 to produce an intermediate product gas 16.

The upstream catalyst is catalytically active for the endothermic (soft oxidant) conversion of the $C_2$-$C_4$ alkane 10 to its corresponding $C_2$-$C_4$ alkene. Carbon dioxide 12 may be supplied to the endothermic reaction zone 14 in any manner known to persons of ordinary skill in the art. For example, as shown in Figure 1, carbon dioxide 12 may be provided as a separate stream directly to the endothermic reaction zone 14, simultaneously with the $C_2$-$C_4$ alkane 10. Other options, which are not shown here, include, but are not limited to: blending the carbon dioxide 12 with the $C_2$-$C_4$ alkane 10 before entry into the endothermic reaction zone 14, or blending the carbon dioxide 12 with one or more other feed streams to the endothermic reaction zone 14.

One or more inert materials, or diluents 13, may also be provided to the endothermic reaction zone 14, separately or mixed with either, or both, of the $C_2$-$C_4$ alkane 10 and carbon dioxide 12. Suitable diluents include, but are not limited to nitrogen, noble gases and steam. The feed composition to the endothermic reaction zone 14 may be, for example, 10-80vol% $C_2$-$C_4$ $C_2$-$C_4$ alkane, 10-50vol% carbon dioxide, and the remainder nitrogen, based upon the total volume of the feed materials. Another example of a suitable feed composition for the endothermic reaction zone may be, without limitation, 30-60vol% $C_2$-$C_4$ alkane, 20-50vol% carbon dioxide, and the remainder nitrogen, based upon the total volume of the feed materials.

Suitable operating conditions for endothermic soft oxidant conversion of a $C_2$-$C_4$ alkane are generally known by persons of ordinary skill and are applicable to operation of the endothermic reaction zone. For example, carbon dioxide, the heated mixed product gas comprising unreacted $C_2$-$C_4$ alkane and, optionally, a diluent, may be supplied to the endothermic reaction zone, separately or mixed, at a total gas hourly space velocity (GHSV) of about 500 $hr^{-1}$ to 100,000 $hr^{-1}$. The reaction pressure is typically in the range of from 0.1 to about 10 atm, for example, from 0.8 to 5.0 atm, and the reaction temperature is typically maintained between 300°C and 900°C, for example, between 450°C and 700°C. Contact time between the reactants and catalyst is typically in the range of from 36 ms (100,000 h$^{-1}$) to 7.2 seconds (500 h$^{-1}$), such as, for example, from 200 ms to 5 seconds. The molecular ratio of unreacted $C_2$-$C_4$ alkane to mild oxidant, such as carbon dioxide, supplied to the exothermic reaction zone may, for example, be in a range of from 1:0.1 to 1:10, or even between 1:1 and 1:5.

At least a portion of the $C_2$-$C_4$ alkane is converted, by soft oxidant conversion in the endothermic reaction zone 14 in the presence of the soft oxidant, to produce an intermediate product gas 16 which comprises at least the corresponding $C_2$-$C_4$ alkene and hydrogen. The intermediate product gas 16 may also comprise one or more of the following compounds: unreacted $C_2$-$C_4$ alkane, oxygen, unreacted carbon dioxide, as well as other compounds including, but not limited to, carbon monoxide and water vapor.

With reference still to Figure 1, the process of the present invention further comprises contacting the intermediate product gas 16 and oxygen 18, with a downstream catalyst (not shown per se) in an exothermic reaction zone 20. The oxygen 18 may be provided in the form of an oxygen-containing gas, and it conveniently provided in the form of air.

The downstream catalyst is catalytically active for the selective combustion of hydrogen to form water and heat. Oxygen 18 may be supplied to the exothermic reaction zone 20 in any manner known to persons of ordinary skill in the art. For example, as shown in Figure 1, oxygen 18 may be provided as a separate stream directly to the exothermic reaction zone 20, simultaneously with the intermediate product gas 16. As another option, not shown here, the oxygen 18 may be blended with the intermediate product gas 16 before entry into the exothermic reaction zone 20.

One or more inert materials, or diluents 19, may also be provided to the exothermic (SHC) reaction zone 20, separately or mixed with either, or both, of the intermediate product gas 16 and oxygen 18. Suitable diluents include, but are not limited to nitrogen, noble gases and steam. The feed composition to the exothermic reaction zone 20 may be, for example, 10-30 vol% $C_2$-$C_4$ alkene, 0-40 vol% unreacted $C_2$-$C_4$ alkane, 10-60 vol% carbon dioxide, 1-15 vol% oxygen, and the remainder nitrogen, based upon the total volume of the feed materials. Another example of a suitable feed composition for the exothermic reaction zone 20 may be, without limitation, 5-20 vol% $C_2$-$C_4$ alkene, 0-30 vol% unreacted $C_2$-$C_4$ alkane, 30-60 vol% carbon dioxide, 5-15 vol% oxygen, and the remainder nitrogen, based upon the total volume of the feed materials.

At least a portion of the hydrogen in the intermediate product gas 16 (i.e., the hydrogen formed during soft oxidant conversion of the $C_2$-$C_4$ alkane) is converted (i.e., combusted, oxidized), in the exothermic reaction zone 20, to produce a cumulative product gas 22 comprising at least the corresponding $C_2$-$C_4$ alkene, water and heat. The cumulative product stream 22 may also comprise one or more of the following compounds: unreacted $C_2$-$C_4$ alkane, unreacted oxygen, unreacted hydrogen, unreacted carbon dioxide, as well as other compounds including, but not limited to, carbon monoxide and nitrogen.

Suitable operating conditions for exothermic selective hydrogen combustion are generally known by persons of ordinary skill and are applicable to operation of the exothermic reaction zone 20. For example, oxygen, the intermediate product gas and, optionally, a diluent, may be supplied to the exothermic reaction zone, separately or mixed, at a total gas hourly space velocity (GHSV) of about (GHSV) of about 1,000 hr$^{-1}$ to 100,000 hr$^{-1}$. The reaction pressure suitable for the exothermic reaction zone 20 is typically in the range of from 0.1 to about 5 atm, for example, from 0.5 to 2.0 atm, and the reaction temperature is typically maintained between 100°C and 500°C. The molecular ratio of hydrogen (or $C_2$-$C_4$ alkene) to oxygen, supplied to the exothermic reaction zone may, for example, be in a range of greater than zero and less than 1.0.

At least a portion of the heat present is separated and recovered from the cumulative product stream 22 and provided to the endothermic reaction zone 16 to provide heat for the catalytic endothermic soft oxidant conversion of the $C_2$-$C_4$ alkane 10. The way that separation of the heat from the cumulative product stream 22 is accomplished is not critical to the invention and may be accomplished in any way known, now or in the future, to persons of ordinary skill in the art. For example, without limitation, one or more heat exchangers 24, such as shell & tube heat exchangers, plate & frame heat exchangers, and air-cooled heat exchangers may be employed to remove heat from the cumulative product gas 22. Removal of heat forms a cooled cumulative product gas 22' which comprises at least the corresponding $C_2$-$C_4$ alkene and no more than no more than about 5% by weight of hydrogen, based on the total weight of the cumulative product gas.

Although not shown in Figure 1, it will be readily recognized by persons of ordinary skill that the cooled cumulative product stream 22' may be subjected to further processing and/or participate in additional reactions. For example, the cooled cumulative product stream 22 may be subjected to one or more interim treatments (not shown), such as, without limitation, drying, compression, and stripping (not shown), to purify the desired corresponding $C_2$-$C_4$ alkene product by separating at least a portion of unreacted reactants and other compounds from the cooled cumulative product stream 22' and produce an oxidation feed stream 26 of suitable composition for providing to partial oxidation reaction zones 26, 28, as described in further detail hereinafter.

Determination of the quantities and how to supply the reactant materials ($C_2$-$C_4$ alkane 10, carbon dioxide 12, oxygen 18, etc.) to each of the endothermic and exothermic reaction zones 14, 20 is well within the ability of persons of ordinary skill in the art, based upon the knowledge generally available as well as the particular reactions, the desired products, and the catalysts selected for use in the reaction zones. For example, where carbon dioxide is expected to interfere with the performance of the selected downstream catalyst, then the carbon dioxide should be provided to the endothermic reaction zone 14 in stoichiometric amounts with the $C_2$-$C_4$ alkane.

As shown in Figure 1, the endothermic and exothermic reaction zones 14, 20 may be contained in a single reactor **SOC/SHC** (shown in phantom), which may be any suitable reactor known in the art including, but not limited to, a batch reactor, a stirred tank reactor, a continuous stirred tank reactor (CSTRs), a tubular reactor, a shell-and-tube heat exchanger reactor, a multiple-pass reactor, a reactor having microchannels, a short contact time reactor, a catalytic fixed bed reactor, and a reactor having a combination of the foregoing features. Each reaction zone 14, 20 may, instead, be disposed within separate reactors (not shown), and various combinations of reactors and reaction zones may be arranged. Each reaction zone 14, 20 may or may not include sub-zones (also not shown), which differ by operating temperature, or catalyst composition, or catalyst concentration, or in other ways which are known to persons of ordinary skill. Furthermore, the upstream and downstream catalysts may be configured in their respective reaction zones in any suitable arrangement including, but not limited to, a fixed bed, a fluidized bed, and a spouted bed. All such configurations are well known in the art.

The cooled cumulative product stream 22' is next used as an oxidation feed stream 26 and comprises the corresponding $C_2$-$C_4$ alkene (e.g., propene), water, and no more than about 5% by weight of hydrogen. With reference back to Figure 1, the oxidation feed stream 26 is fed, sequentially, to the partial oxidation reaction zones PO, 28, 30. In particular, as shown schematically in Figure 1, the oxidation feed stream 26, along with an oxygen-containing gas 32 and, optionally, steam 34, is provided to the first 28 of two oxidation reaction zones 28, 30. Addition of oxygen-containing gas to the starting materials provides molecular oxygen to the reaction system. It is usually most economical to use an oxygen-containing gas such as air, since purity is not particularly required.

Any type of reactors that are suitable for performing the desired vapor phase oxidation reactions may be used to contain, or hold, the oxidation reaction zones 28, 30. Shell-and-tube reactors, for example and without limitation, are suitable for use in this regard.

Suitable diluting gases 35 may, optionally, also be provided to the first oxidation reaction zone 28, including, but not limited to, one or more of: carbon monoxide, carbon dioxide, or mixtures thereof, an inert gas, such as nitrogen, argon, helium, or mixtures thereof. A suitable molar ratio of the starting materials for the total feed materials to the first oxidation reaction zone 28, ($C_2$-$C_4$ alkane, $C_2$-$C_4$ alkene, or a mixture thereof) : (oxygen): ($H_2O$) : (diluting gas), would be, for example, (1) : (0.1 to 10) : (0 to 20) : (0.2 to 70), for example, including but not limited to, (1) : (1 to 5.0) : (0 to 10) : (5 to 40). Where it is desired to produce unsaturated carboxylic acids, it is beneficial to include steam 34 among the starting materials, as shown in Figure 1 and mentioned hereinabove. The steam to be employed may be present in the form of steam gas in the reaction system, and the manner of its introduction is not particularly limited.

When steam 34 is supplied together with the mixture of $C_2$-$C_4$ alkanes and alkenes, as starting material gas, the selectivity for an unsaturated carboxylic acid is distinctly improved, and the unsaturated carboxylic acid can be obtained in good yield. However, the conventional technique utilizes a diluting gas, as described above, for the purpose of diluting the starting material. Such a diluting gas is used to adjust the space velocity, the oxygen partial pressure and the steam partial pressure, as will be readily understood by persons having ordinary skill in the art.

If it is desired to produce unsaturated nitriles from the corresponding $C_2$-$C_4$ alkanes, ammonia (not shown) must also be provided to the first oxidation reaction zone 28. In such cases, the ammonia may be provided simultaneously, but separately from the oxidation feed stream 26, or any of the other feed streams 32, 34. Alternatively, the ammonia may be combined with any one or more of the other feed streams 26, 32, 34 to the first oxidation reaction zone 28.

With reference now to the oxidation reaction zones 28, 30, one or more oxidation catalysts (not shown, per se) are contained within each oxidation reaction zone 28, 30. The oxidation catalysts are capable of facilitating the desired vapor phase oxidation reaction, in this case, partial oxidation of a $C_2$-$C_4$ alkene (e.g., propene) to the corresponding unsaturated carboxylic acid (e.g., acrylic acid). The oxidation catalysts may be of different configuration, including but not limited to fixed-bed, moving-bed and fluidized-bed. In addition, any suitable catalyst may be used and would be selected based upon the particular $C_2$-$C_4$ alkane, $C_2$-$C_4$ alkene, or mixture thereof, and the desired oxidation products. As is well known in the art, the catalysts may be used alone, or they may also be used together with a carrier, or support, such as, without limitation, silica, alumina, titania, aluminosilicate or diatomaceous earth. Further, depending upon the scale or system of the reaction, they may be molded into a proper shape and/or particle size. The particular shape or geometry of the catalysts are not particularly limited in connection with the present invention.

The selection of the catalysts, their shape, size and packing method, are well within the ability of persons having ordinary skill in the art. In general, any catalysts that are capable of catalyzing the two-step vapor phase catalytic oxidation of a reactive hydrocarbon to an unsaturated aldehyde or acid are suitable for use in connection with the present invention. For example, one embodiment of the multiple catalyst system of the present invention is capable of catalyzing propene to acrylic acid. Although the following discussion will describe the multiple catalyst system as applied to the production of acrylic acid from propene by two-step vapor phase catalytic oxidation, it will be understood, as previously described, that the present invention is not limited to such application and is suitable for use in the production of other unsaturated aldehydes and acids from other types of reactive hydrocarbons.

Catalysts capable of oxidizing an alkene to the corresponding aldehyde are hereinafter referred to as "first oxidation catalysts" and are suitable for use with the present invention, as will be discussed in further detail hereinafter. In addition, catalysts capable of oxidizing the aforesaid aldehyde to the corresponding unsaturated carboxylic acid "second oxidation catalysts". Either the first oxidation catalysts, or the second oxidation catalysts, or both, are typically, but need not be, mixed metal oxide compositions.

For example, the first oxidation reaction zone 28 may contain a first oxidation catalyst and the second oxidation reaction zone 30 may contain a second oxidation catalyst. Other embodiments are possible wherein the multiple catalyst system comprises more than one type of first oxidation catalyst and a second oxidation catalyst, or alternatively, a first oxidation catalyst and more than one type of second oxidation catalyst. Furthermore, the catalysts contained in the first and second oxidation reaction zones 28, 30 may comprise more than one type of first oxidation catalyst and more than one type of second oxidation catalyst. The foregoing variations, and others, will be readily apparent to persons of ordinary skill in the art based upon the following description.

In particular, a first oxidation catalyst suitable for use in connection with the present invention comprises at least one atom of a group VIB element, at least one atom of a group VA element, and at least two group VIII atoms and is capable of catalyzing the production of an aldehyde, such as acrolein. For example, the first oxidation catalyst may contain more than one atom of the same group VIII element (e.g., group VIII atoms include Fe, Co, Ni, Ru, Rh, Pd, Os, Ir, Pt). In another embodiment, the R1 catalyst comprises two group VIII atoms which are different elements. In a further embodiment, the first oxidation catalyst comprises at least Fe, Co or Ni. Yet another embodiment of first oxidation catalyst includes at least two different atoms selected from Fe, Co or Ni and compounds which are mixture thereof (e.g., FeCo, FeNi, CoFe, CoNi).

Commonly, the first oxidation catalyst comprises at least one atom of a group VIB element (e.g., Cr, Mo, W, or Unh), for example, at least Mo. In one embodiment, the first oxidation catalyst comprises at least one atom of a group VA element (e.g., N, P, As, Sb or Bi), and in another embodiment, the catalyst comprises Mo, Bi, Fe and at least one atom of Ni or Co.

For example, suitable catalysts for the oxidation of propene to acrolein are described in, for instance, U.S. Patent Nos. 4,025,565; 5,821,390; and 5,929,275.

A second oxidation catalyst suitable for use in connection with the present invention may comprise at least one atom of a group VIB element (e.g., Cr, Mo or W), at least one atom of a group VB element (e.g., V, Nb or Ta), at least one atom of a group IB element (e.g., Cu, Ag, or Au), and a group VA element (e.g., N, P, As, Sb or Bi). For example, the second oxidation catalyst may comprise one or more atoms of elements from groups including: IA (alkali metal element, e.g., H, Li, Na, K, Rb, Cs, or Fr); IIA (alkali earth metal element, e.g., Be, Mg, Ca, Sr or Ba); IIIB which also encompasses elements of the lanthanide series and the actinide series (e.g., Sc, Y, La, Ce, Pr, Nd, Pm, Sm, Eu, Gd, Yb or U); IVB (e.g., Ti, Zr or Hf); VB (e.g., V, Nb or Ta); VIIB (e.g., Mn, Tc and Re); VIII (e.g., Fe, Co, Ni, Ru, Rh, Pd, Os, Ir, Pt); IIB (e.g., Zn, Cd, or Hg); IIIA (e.g., B, Al, Ga or In); IVA (e.g., C, Si, Ge, Sn, or Pb); VA (e.g., N, P, As, Sb, or Bi); or VIA (e.g., S, Se or Te).

In one embodiment, the second oxidation catalyst comprises Mo, V, Cu, W and Sb. Another embodiment, the second oxidation catalyst comprises antimony present in an amount less than about 5 wt % with a concentration of less than 3% typical and less than 1% in another embodiment. In still another embodiment, the second oxidation catalyst comprises $MoO_2$ and $MoO_3$ either individually (i.e., $MoO_2$ without $MoO_3$; or $MoO_3$ without $MoO_2$) or in combination (i.e., $MoO_2$ and $MoO_3$).

Suitable catalysts for the oxidation of acrolein to acrylic acid, for example, without limitation, are described in U.S. Patent Nos. 3,775,474; 3,893,951; 3,954,855; 4,075,127; 4,146,732; 4,259,211; 4,339,355; 5,177,260; and 5,739,391.

With reference still to Figure 1, In the first oxidation reaction zone 28, the oxidation feed materials 26, 32, 34 come into contact with the first oxidation catalyst therein and react with one another to form desired oxidation products, as well as various side products and by-products, according to the particular types of $C_2$-$C_4$ alkanes and alkenes used. When the oxidation feed stream 26 comprising $C_2$-$C_4$ alkene contacts the one or more catalysts contained in the first oxidation reaction zone, an intermediate oxidation effluent stream 36 is formed, which comprises at least the corresponding aldehyde, along with unreacted alkane, unreacted alkene, carbon dioxide and oxygen. For example, without limitation, where the $C_2$-$C_4$ alkene in the oxidation feed stream 26 comprises propene, the corresponding aldehyde in the effluent stream 36 will comprise acrolein.

As shown in Figure 1, the intermediate oxidation effluent stream 36 is fed into the second oxidation reaction zone 30, along with additional oxygen-containing gas, such as air 38, and, optionally, additional steam 40. More particularly, the additional oxygen-containing gas 38 may be first combined with the intermediate oxidation effluent stream 36 and then fed together, as a combined stream (not shown) to the second oxidation reaction zone 30. Alternatively, the additional oxygen-containing gas 38 may be fed to the second oxidation reaction zone 30 as a separate feed stream (see Figure 1).

In the second oxidation reaction zone 30, the corresponding aldehyde and oxygen come into contact with a second oxidation catalyst situated in the second oxidation reaction 30 and react with one another to form the desired oxidation products, as well as various side products and by-products, according to the particular types of alkanes and alkenes used. A final oxidation effluent stream 42 (see Figure 1) exits the second oxidation reaction zone 30 and typically contains, but is not limited to, one or more oxidation products (e.g., unsaturated carboxylic acid), unreacted oxygen, and unreacted $C_3$ to $C_5$ alkane or alkene, or mixture thereof, as well as reaction by-products which may include, but are not limited to, acetic acid and carbon dioxide and, possibly, unreacted water and unreacted ammonia, depending upon the starting materials used. The final oxidation effluent 42 may be fed to additional processing apparatus (not shown) to undergo separation and purification processes, as is well-known to persons having ordinary skill in the art, to recover the one or more oxidation products.

For example, as will be described in greater detail below in connection with the Examples, the final oxidation effluent stream 42 may be subjected to quenching with water in an absorber, followed by one or more separations steps to remove impurities and concentrate the acrylic acid product. Furthermore, in one embodiment, the gas from the absorber may be dried, such as in a molecular sieve dryer, and then fed to a pressure swing adsorption system to recover unreacted propane and carbon dioxide for recycle back to the upstream soft oxidant conversion reaction zone. In another embodiment, pressure swing adsorption is omitted and the dried as from the absorber is simply recycled back to the soft oxidant conversion reaction zone, with the recycle amount and composition being such that separation of unreacted propane and carbon dioxide is unnecessary.

It will be understood that the embodiments of the present invention described hereinabove are merely exemplary and that a person skilled in the art may make variations and modifications without departing from the spirit and scope of the invention. All such variations and modifications are intended to be included within the scope of the present invention.

**EXAMPLES**

**Example 1**

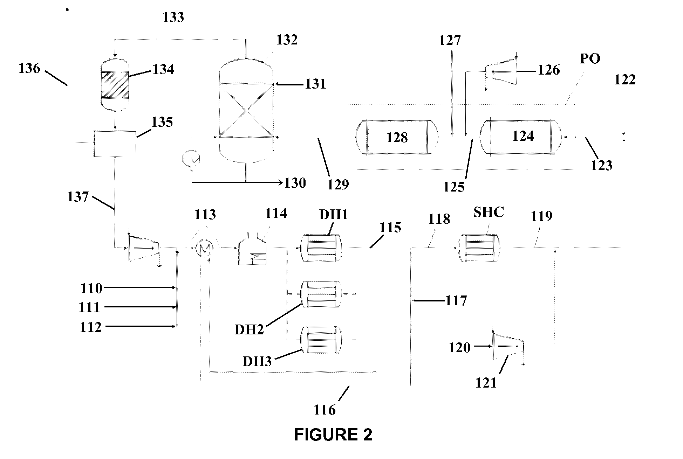

The following description refers to the schematic representation of process equipment shown in Figure 2. Three soft oxidant conversion reactors (SOC1, SOC2, SOC3), each comprising one oxidation reaction zones (not shown per se, but see Figure 1 and accompanying description above), are loaded with suitable catalysts (also not shown per se, but see Figure 1 and accompanying description above). One reactor at a time is in operation and is used to convert the propane to propene using thermally integrated soft oxidant conversion processes. When one reactor is not in use, i.e., "off-line," it undergoes catalyst regeneration. The soft oxidant conversion reactors (SOC1, SOC2, SOC3) are automatically cycled on-line and off-line by a process control system (not shown).

The fresh (i.e., not including any recycled materials) starting materials fed to the soft oxidant conversion reaction zones are: propane (110) at 13140 kg/hr, carbon dioxide (111) at 8573 kg/hr, and nitrogen (112) at 5287 kg/hr. Recycle gas (137), at 62340 kg/hr and comprising propane and carbon dioxide, is also fed to the soft oxidant conversion reaction zones, in addition to the fresh starting materials. Each of the propane, carbon dioxide, nitrogen, and recycle streams (110, 111, 112, 137) has the compositions listed in the following Table 1:

Table 1: Feed Composition to Soft Oxidant Conversion (SOC) Reaction Zones

| Component | % (by volume) | | | |
| --- | --- | --- | --- | --- |
| | Propane | Nitrogen | Carbon Dioxide | Recycle |
| Propane | 100 | | | 40 |
| Nitrogen | | 100 | | |
| Carbon Dioxide | | | 100 | 60 |

In this particular application, the recycle gas (137) is compressed to 2.6 bar and combined with the propane, $CO_2$ and nitrogen (110, 111, 112). The combined propane feed stream (113) is heated by exchange with the soft oxidant conversion reactor effluent (115) to 525°C and then in a feed heater (114) to the reaction temperature, 625°C.

Each soft oxidant conversion reactor is loaded with a catalyst containing 10% $Cr_2O_3$ supported on Merck 10181 for conversion of propane in the presence of a weak oxidant (the carbon dioxide), to produce propene and hydrogen. The contact time is 0.50 sec*ml/g. Heat from the selective hydrogen combustion reactor (SHC) and heat from the regeneration step of the soft oxidant conversion reactors (SOC1, SOC2, SOC3) can be used to offset the energy requirements of the soft oxidant conversion reaction to obtain a heat neutral balance. The composition of the effluent gas (115) from the soft oxidant conversion reaction zones is provided in the following Table 2:

Table 2: Product Stream Composition from Soft Oxidant Conversion Reaction Zones

| Component | % (by volume) |
|---|---|
| Carbon Dioxide | 38 |
| Carbon Monoxide | 6 |
| Ethylene | 1 |
| Hydrogen | 7 |
| Methane | 2 |
| Nitrogen | 8 |
| Propane | 24 |
| Propene | 10 |
| Water | 6 |

To the cooled effluent gas (116) is combined with 2767 kg/hr oxygen (117). The combined stream (118) is fed to the selective hydrogen combustion reactor (SHC). The reactor is loaded with a suitable supported platinum-based catalyst. The composition of the effluent gas (119) from the SHC is provided in the following Table 3:

Table 3: Product Stream Composition from Selective Hydrogen Combustion

| Component | % (by volume) |
|---|---|
| Carbon Dioxide | 37 |
| Carbon Monoxide | 6 |
| Ethylene | 1 |
| Hydrogen | 1 |
| Methane | 2 |
| Nitrogen | 8 |
| Propane | 23 |
| Propene | 10 |
| Water | 12 |

Oxygen for the partial oxidation reaction in the next reactor (124) can be supplied with from either air (120) or pure oxygen (122). The effluent (119) from the SHC is combined with 11822 kg/hr oxygen. The first propene oxidation reactor feed stream (123) is fed to the first (124) of two partial oxidation reaction zones (PO), at a rate of 104774 kg/hr, and has the composition listed in the following Table 4:

Table 4: Feed Stream Composition to Partial Oxidation Reaction Zones_

| Component | % (by volume) |
|---|---|
| Carbon Dioxide | 32.2 |
| Carbon Monoxide | 4.8 |
| Ethylene | 1.0 |
| Hydrogen | 1.1 |
| Methane | 1.5 |
| Nitrogen | 10.1 |
| Oxygen | 13.9 |
| Propane | 20.1 |

(continued)

| Component | % (by volume) |
|---|---|
| Propene | 8.7 |
| Water | 6.6 |

Propene is converted to acrolein in the first partial oxidation reaction zone (124), in the presence of a mixed metal oxide catalyst containing Mo-W-Bi-Fe-Co-Cs-Si (not shown per se). The acrolein product stream (125) from the first partial oxidation reaction zone (124) is combined with air (125) at 167 $m^3$/min (STP) and steam (127) at 115 kg/hr and fed to a second partial oxidation reaction zone (128), wherein the acrolein is converted to acrylic acid, in the presence of a mixed metal oxide catalyst containing as essential compounds Mo-V-W-Cu-Sb-Ti (not shown per se). The composition of the gaseous effluent stream (129) from the second partial oxidation reaction zone (128) is as follows:

Table 5: Effluent Stream Composition from Partial Oxidation Reaction Zones

| Component | % (by volume) |
|---|---|
| Acetaldehyde | 0.03 |
| Acetic Acid | 0.1 |
| Acrylic Acid | 7.4 |
| Acrolein | 0.04 |
| Carbon Dioxide | 32.9 |
| Nitrogen | 18.9 |
| Oxygen | 3.7 |
| Propane | 19.8 |
| Propene | 0.2 |
| Water | 17.0 |

The effluent (129) from the partial oxidation reaction zones (PO) is quenched with water (131) in an absorber (132). The aqueous, crude acrylic acid (130) is conducted to separations apparatus (not shown) to concentrate the acrylic acid and remove impurities. The gas (133) from the absorber (132) is dried in a molecular sieve dryer (134) and fed to a pressure swing adsorption system (135) to separate and recover unreacted propane and $CO_2$. In order to meet feed concentration requirements for the ODH reactor, propane and $CO_2$ need to be separated and recovered from them gas from the absorber. Without such recovery, a significant portion of the gas from the absorber could no be used for recycle and would need to be discarded. The pressure swing adsorption system (135) is assumed to recover 99% of the unreacted propane and 90% of the $CO_2$. The recovered propane and $CO_2$ (137) are recycled to the soft oxidant conversion reactors (SOC1, SOC2, SOC3). The non-recycled material (136) can be incinerated or used as a fuel source.

## Claims

1. A process for producing an unsaturated carboxylic acid or an unsaturated nitrile from the corresponding $C_2$-$C_4$ alkane, said process comprising the steps of:

   A) converting a $C_2$-$C_4$ alkane to its corresponding $C_2$-$C_4$ alkene by providing a $C_2$-$C_4$ alkane, a weak oxidant and heat to an endothermic reaction zone comprising an upstream catalyst to produce an intermediate product gas comprising at least the corresponding $C_2$-$C_4$ alkene and hydrogen;
   B) converting at least a portion of the hydrogen in the intermediate product gas to water by providing the intermediate product gas and oxygen to an exothermic reaction zone to produce a cumulative product gas which comprises the corresponding $C_2$-$C_4$ alkene, water, carbon oxides and heat;
   C) recovering at least a portion of the heat from the cumulative product gas and providing the recovered heat to the endothermic reaction zone, wherein at least a portion of the heat provided in step A) comprises the recovered heat and wherein a cooled cumulative product gas is produced;

D) oxidizing the $C_2$-$C_4$ alkene of the cooled cumulative product gas to produce an oxidation product stream comprising the corresponding unsaturated carboxylic acid or nitrile, in the presence of at least one catalyst capable of facilitating the oxidizing reaction;

E) separating the oxidation product stream to form an aqueous oxidation product stream comprising the corresponding unsaturated carboxylic acid or nitrile, and a gas stream comprising unreacted $C_2$-$C_4$ alkane and unreacted carbon dioxide;

F) drying the gas stream and separating the unreacted $C_2$-$C_4$ alkane and unreacted carbon dioxide therefrom; and

G) recycling the separated unreacted $C_2$-$C_4$ alkane and unreacted carbon dioxide to the step A) of converting a $C_2$-$C_4$ alkane in an endothermic reaction zone.

2. The process of Claim 1, wherein the weak oxidant comprises carbon dioxide.

3. The process of Claim 1, wherein the upstream catalyst is a soft oxidant conversion catalyst which catalyzes the endothermic conversion of the $C_2$-$C_4$ alkane to the corresponding $C_2$-$C_4$ alkene in the presence of the weak oxidant.

4. The process of Claim 1, wherein the downstream catalyst is a selective hydrogen combustion catalyst which catalyzes the exothermic conversion of hydrogen to water in the presence of oxygen.

5. The process of Claim 3, wherein the soft oxidant conversion catalyst comprises:

A) chromium or chromium oxide;
B) optionally, one or more metals selected from the group consisting of Mo, W, V, Ga, Mg, Ni and Fe; and
C) optionally, one or more metals selected from the group consisting of Ag, V and Ga.

6. The process of Claim 3, wherein the soft oxidant conversion catalyst comprises a support material.

7. The process of Claim 4, wherein the selective hydrogen combustion catalyst comprises at least one catalyst composition selected from the group consisting of:

A) a catalyst comprising a noble metal selected from the group consisting of platinum and palladium, and, optionally, another metal selected from the group consisting of tin and iridium; and
B) a catalyst comprising an oxide of indium or bismuth, and, optionally, another metal selected from the group consisting of molybdenum.

8. The process of Claim 7, wherein the selective hydrogen combustion catalyst comprises a support material.

9. The process of Claim 1, wherein the $C_2$-$C_4$ alkane comprises propane, the corresponding $C_2$-$C_4$ alkene comprises propene, the weak oxidant comprises carbon dioxide, and the corresponding unsaturated carboxylic acid is acrylic acid.

10. The process of Claim 1, wherein the oxidizing step H) is performed by a two-step vapor phase catalytic oxidation wherein a first oxidation reaction zone comprises a first oxidation catalyst capable of catalyzing the conversion of the $C_2$-$C_4$ alkene to the corresponding aldehyde, and a second oxidation reaction zone, positioned downstream of the first oxidation reaction zone and comprising a second oxidation catalyst, different from the first oxidation catalyst and capable of catalyzing the conversion of the corresponding aldehyde to the corresponding unsaturated carboxylic acids or nitriles.

11. The process of Claim 1, wherein the separating step I) is accomplished by quenching the oxidation product stream with water in an absorber.

12. The process of Claim 1, wherein separating the unreacted $C_2$-$C_4$ alkane and unreacted carbon dioxide from the gas stream from the absorber is performed by using a pressure swing absorption system.

13. The process of Claim 1, wherein the cooled cumulative product gas further comprises no more than about 5% by weight of hydrogen, based on the total weight of the cooled cumulative product gas.

**Figure 1**

**FIGURE 2**

Europäisches
Patentamt

European
Patent Office

Office européen
des brevets

# EUROPEAN SEARCH REPORT

**Application Number**

EP 09 15 5567

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2006/004226 A1 (MACHHAMMER OTTO [DE] ET AL MACHHAMMER OTTO [DE] ET AL) 5 January 2006 (2006-01-05) | 1-12 | INV. C07C51/25 C07C57/055 C07C253/26 |
| Y | * the whole document * | 13 | |
| Y,D | TAKEHIRA K ET AL: "CO2 dehydrogenation of propane over Cr-MCM-41 catalyst" STUDIES IN SURFACE SCIENCE AND CATALYSIS, ELSEVIER SCIENCE B.V., AMSTERDAM, NL, vol. 153, 1 January 2004 (2004-01-01), pages 323-328, XP009116376 ISSN: 0167-2991 * the whole document * | 13 | |

-----

-----

| | | | TECHNICAL FIELDS SEARCHED (IPC) |
|---|---|---|---|
| | | | C07C |

The present search report has been drawn up for all claims

| Place of search | Date of completion of the search | Examiner |
|---|---|---|
| Munich | 17 September 2009 | Seelmann, Marielle |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

.................................................................................

& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 09 15 5567

This annex lists the patent family members relating to the patent documents cited in the above-mentioned European search report.
The members are as contained in the European Patent Office EDP file on
The European Patent Office is in no way liable for these particulars which are merely given for the purpose of information.

17-09-2009

| Patent document cited in search report | Publication date | Patent family member(s) | Publication date |
|---|---|---|---|
| US 2006004226 A1 | 05-01-2006 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 0117146 A
- US 5705684 A
- US 4046833 A
- US 6239325 A
- US 20050124840 A
- US 20050085678 A
- EP 1112241 A
- US 4788371 A
- US 11901102 B
- US 61009118 B
- US 4025565 A
- US 5821390 A
- US 5929275 A
- US 3775474 A
- US 3893951 A
- US 3954855 A
- US 4075127 A
- US 4146732 A
- US 4259211 A
- US 4339355 A
- US 5177260 A
- US 5739391 A

### Non-patent literature cited in the description

- **Zhagorigetu, B. ; Kieffer, R. ; Hinderman J.-P.** Oxidative Dehydrogenation of Propane on Rare Earth Vanadates. Influence of the Presence of CO2 in the feed. *Studies in Surface Science and Catalysis,* 1996, vol. 101, 1049-1058
- **Li, X.-H. ; Li, W.-Y. ; Xie, K.-C.** Supported Vanadia Catalysts for Dehydrogenation of Ethylbenzene with CO2. *Catalyst Letters,* December 2005, vol. 105 (3-4
- **Dury, F. ; Gaigneaux, E.M. ; Ruiz, P.** The Active Role of CO2 at Low Temperature in Oxidation Processes: The Case of the Oxidative Dehydrogenation of Propane on NiMoO4 catalysts. *Applied Catalysis A: General,* 2003, vol. 242, 187-203
- **Takehira, K. ; Oishi, Y. ; Shishido, T. ; Kawabata, T. ; Takaki, K. ; Zhang, Q. ; Wang, Y.** CO2 Dehydrogenation of Propane over Cr-MCM-41 Catalyst. *Studies in Surface Science and Catalysis,* 2004, vol. 153, 323-328
- **Grasselli et al.** Catalytic dehydrogenation (DH) of light parrafins combined with selective hydrogen combustion (SHC) I. DH -> SHC -> DH catalysts in series (co-fed process mode). *Applied Catalysis A: General,* 1999, vol. 189, 1-8
- **Grasselli et al.** Catalytic dehydrogenation (DH) of light parrafins combined with selective hydrogen combustion (SHC) II. DH+SHC catalysts physically mixed (redox process mode). *Applied Catalysis A: General,* 1999, vol. 189, 9-14